# EUROPEAN PATENT APPLICATION

(11) **EP 4 410 834 A1**
(43) Date of publication of application: **07.08.2024**
(21) Application number: 22875001.4
(22) Date of filing: 28.09.2022
(51) Int. Cl.: C07K 16/28, C12N 15/13, C12N 15/63, C12P 21/02, A61K 39/395, A61P 35/00, G01N 33/68, G01N 33/574

(54) **CTLA-4 BINDING MOLECULE AND USE THEREOF**

(30) Priority: 29.09.2021 CN 202111152925
(71) Applicant: Zhejiang Nanomab Technology Center Co. Ltd., Changle Township Shengzhou Shaoxing City Zhejiang Province, 312467 (CN)
(72) Inventor: LI, Jiaguo, Shanghai 201805 (CN); ZHU, Weimin, Danville, California 94506 (US); SUN, Yan, Shanghai 201805 (CN); QIAN, Qijun, Shanghai 201805 (CN)
(74) Representative: van Dam, Vincent
(86) International application number: PCT/CN2022/122178
(87) International publication number: WO 2023/051618

(57) **Abstract**

The present invention relates to a CTLA-4 binding molecule and the use thereof. The CTLA-4 binding molecule of the present invention contains an anti-CTLA-4 single domain antibody, wherein the complementary determining region CDR of the single domain antibody comprises CDR1, CDR2 and CDR3, CDR1 comprises a sequence as shown in SEQ ID NO: 1, CDR2 comprises a sequence as shown in SEQ ID NO: 2, and CDR3 comprises a sequence as shown in SEQ ID NO: 3. Further provided in the present invention are a sequence encoding the CTLA-4 binding molecule, an expression vector, and a corresponding host cell, pharmaceutical composition and use. The CTLA-4 binding molecule of the present invention can bind to an extracellular region of CTLA-4 with a high specificity, and has a high affinity and biological activity, a low immunogenicity, a stable structure and a good druggability.

## Description

### Technical Field

The invention relates to the biomedical or biopharmaceutical technology field, more specifically to a cytotoxic T-lymphocyte associated antigen 4 (CTLA-4) binding molecule and its application. The CTLA-4 binding molecule of the invention can bind to the extracellular region of CTLA-4 with high specificity and high affinity, resulting in high biological activity, and has low immunogenicity, stable structure and good druggability.

### Background

T cell activation requires two signals: the first signal is initiated by recognition of antigen peptide-MHC complex (p-MHC) on the surface of APC with T cell receptor complex (TCR) (Weiss, j.clin.invest.86:1015 (1990)); and the second signal is initiated by the interaction of costimulatory molecules between T cells and APCs (Schwartz, science248:1349 (1990)). The most important costimulatory molecule pair is the binding between B7 molecules expressed on the surface of professional APCs and the dimeric molecule CD28 on the surface of T cells. CD28 transmits signals to T cells to initiate T cell responses, including promoting the production of IL-2 molecules and the differentiation of naive T cells into effector T cells and memory T cells.

Cytotoxic T-lymphocyte associated antigen 4 (CTLA-4) and CD28 are co stimulatory receptors on the surface of T cells, belonging to the B7 molecule family. CTLA-4 molecule is also a member of the immunoglobulin (Ig) superfamily, containing a single extracellular Ig domain. CTLA-4 contains six amino acid sequences MYPPPY, and has 70% homology with the molecular structure of CD28, both of them binding to the same ligands, namely B7-1 (CD80) and B7-2 (CD86). However, the role of CTLA-4 molecule is opposite to that of CD28 molecule. CD28 molecule is expressed on quiescent or active T cells, and promotes activation and cytokine production of T cells upon binding with ligands; while CTLA-4 is only expressed in activated T cells, with a peak expression at about 24 hours after T cell activation, and can hardly be detected in quiescent T cells. It can inhibit T cell proliferation and reduce the production of cytokine IL-2, which is related to the maintenance of immune homeostasis and peripheral tolerance (Linsley PS et al.J exp Med, 1992176 (6):1595-1604; Bour Jordan h et al., immune rev, 2011, 241 (1): 180-205.). CTLA-4 is also found in regulatory T cells (Tregs) and contributes to their suppressive function. Meanwhile, CTLA-4 has higher binding affinity than CD28, which makes it a potential treatment for autoimmune diseases.

In view of the immunosuppressive function of CTLA-4 molecule, blocking CTLA-4 can enhance the activation and proliferation of T cells. At present, the drug development for CTLA-4 molecule mainly focuses on fusion proteins, monoclonal antibodies, bispecific antibodies, etc., which are used to treat autoimmune diseases and tumors respectively. At present, two CTLA-4 fusion proteins and one CTLA-4 antibody have been launched worldwide, namely Orencia (Abatacept) in 2005, Nulojix (Belatacept) and Yervoy (ipilimumab) in 2011. Among them, Abatacept fusion protein was approved for the treatment of rheumatoid arthritis, which can effectively improve symptoms and signs, and delay the progress of joint radiology; Belatacept was approved for the prevention of acute rejection in adult kidney transplant recipients; and Ipilimumab was approved to be combined with nivolumab for the treatment of cancers including melanoma, renal cancer, lung cancer, colorectal cancer and other cancers, showing better anti-tumor effect than single antibody. In terms of bispecific antibodies, Alphamab Oncology developed bispecific antibodies (KN046) against CTLA-4 and PD-L1, which has entered phase I clinical trials in Australia. This antibody has also obtained the phase I clinical approval from the China National Drug Administration, and will enter the clinical trials in China. KN046 was targeted for use in the tumor microenvironment, thereby reducing side effects on the human peripheral system. In preclinical studies, KN046 has shown excellent antitumor efficacy, and its toxicity is significantly reduced compared with the existing CTLA-4 antibody ipilimumab. Ongoing clinical trials have also shown its good tolerability to humans.

Nanobody has the natural advantages of easy expression, high stability and high affinity. According to the advantages of nanobody and the biological mechanism of CTLA-4, the development of CTLA-4 nanobody has a very broad application prospect.

### Summary

The invention aims to provide a novel anti-CTLA-4 binding molecule and use thereof.

The first aspect of the invention provides a CTLA-4 binding molecule, which comprises an anti-CTLA-4 single domain antibody, wherein the complementarity determining region (CDR) of the single domain antibody comprises CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3.

In one or more embodiments, SEQ ID NO: 1 is GX₁X₂X₃X₄X₅X₆X₇, wherein X₁ is S, L, D, F or G, X₂ is T, N or S, and X₃ is L, F or S, X₄ is D, GorE, X₅ is P, I, S, D or Y, X₆ is F, Y, H, Q or W, and X₇ is V, D, A or S.

In one or more embodiments, CDR1 comprises the sequence shown in any one of SEQ ID NOs: 4-10.

In one or more embodiments, SEQ ID NO: 2 is X₁X₂X₃X₄X₅X₆X₇X₈, wherein X₁ is I or M, X₂ is N, G, S or R, X₃ is R, P, S or G, X₄ is S, N, D or T, X₅ is R or G, X₆ is T, A, D, E, R, G or L, X₇ is I, F, T, S, G, R or null, and X₈ is T or null.

In one or more embodiments, CDR2 comprises the sequence shown in any one of SEQ ID NOs: 11-18.

In one or more embodiments, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, G or T, X₂ is A, L, S or G, X₃ is S, G, E, V, A or K, X₄ is the P, R or L, X₅ is D, E, L, T, I or A, X₆ is K, R, V, L, S or H, X₇ is P, A, Y, F, G or Q, X₈ is F, H, T, V or S, X₉ is Y, S, L, A, H or M, X₁₀ is T, I, C, A or W, X₁₁ is D, Y, S, L or V, X₁₂ is Q, A, L, M or C, and X₁₃ is T, S, N or C, and X₁₄ is M, E, H, Q, A or null, X₁₅ is C, Y, G, H, A, N or null, X₁₆ is N, D, M, Y, S, E or null, X₁₇ is K, I, Y, R, F or null, X₁₈ is H, D, S, G or null, and X₁₉ is Y, T, D, L or null, X₂₀ is Y or null, X₂₁ is G or null, and X₂₂ is Y or null.

In one or more embodiments, CDR3 comprises the sequence shown in any one of SEQ ID NOs: 19-26.

In one or more embodiments, CDR1 comprises the sequence shown in any one of SEQ ID NOs: 4-10, CDR2 comprises the sequence shown in any one of SEQ ID NOs: 11-18, and CDR3 comprises the sequence shown in any one of SEQ ID NOs: 19-26.

In one or more embodiments, the single domain antibody comprises CDR1, CDR2, and CDR3 shown in any one of the following groups a1 to a9:

| Group | CDR1 | CDR2 | CDR3 |
|---|---|---|---|
| a1 | 4 | 11 | 19 |
| a2 | 4 | 12 | 20 |
| a3 | 4 | 12 | 20 |
| a4 | 5 | 13 | 21 |
| a5 | 6 | 14 | 22 |
| a6 | 7 | 15 | 23 |
| a7 | 8 | 16 | 24 |
| a8 | 9 | 17 | 25 |
| a9 | 10 | 18 | 26 |

In one or more embodiments, FR1 of the single domain antibody VHH is the FR1 region of any VHH selected from SEQ ID NOs: 27-73, FR2 of VHH is the FR2 region of any VHH selected from SEQ ID NOs: 27-73, FR3 of VHH is the FR3 region of any VHH selected from SEQ ID NOs: 27-73, and FR4 of VHH is the FR4 region of any VHH selected from SEQ ID NOs: 27-73.

In one or more embodiments, the FR region of the single domain antibody is the FR region of any VHH selected from SEQ ID NOs: 27-73.

In one or more embodiments, the single domain antibody VHH is as shown in any one of SEQ ID NOs: 27-73.

In one or more embodiments, the CTLA-4 binding molecule is a monovalent or multivalent single domain antibody, a multispecific single domain antibody, a heavy chain antibody or an antigen binding fragment thereof, an antibody or an antigen binding fragment thereof comprising one, two or more of the anti-PD-1 single domain antibodies described herein.

In one or more embodiments, the multivalent single domain antibody or multispecific single domain antibody connects a plurality of single domain antibodies through a linker. The linker consists of 1-15 amino acids selected from G and S.

In one or more embodiments, the antigen binding fragment of the heavy chain antibody is a single chain heavy chain antibody.

In one or more embodiments, the heavy chain antibody is a camelid heavy chain antibody or a shark heavy chain antibody.

In one or more embodiments, the heavy chain antibody further comprises a heavy chain constant region.

In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG1 or IgG4. Preferably, the heavy chain constant region is CH2 and CH3 of IgG4, and its amino acid sequence is shown in SEQ ID NO: 74.

In one or more embodiments, the heavy chain constant region is a constant region of the shark heavy chain antibody, comprising CH1, CH2, CH3, CH4, and CH5.

In one or more embodiments, the antibody is an antibody comprising the anti-CTLA-4 single domain antibody as the heavy chain variable domain.

In one or more embodiments, the antibody further comprises a light chain variable domain, a heavy chain constant domain, and a light chain constant domain.

In one or more embodiments, the antigen binding fragment of the antibody is selected from Fab, F(ab')2, Fv, scFv.

In one or more embodiments, the binding molecule described in any embodiment of the present description is a chimeric antibody or a fully human antibody; preferably, a fully human antibody.

The description also provides a polynucleotide, comprising a sequence selected from:
(1) a coding sequence of the single domain antibody or the antibody or the antigen binding fragment thereof according to any embodiment herein;
(2) a complementary sequence of (1);
(3) a 5-50bp fragment of any sequence of (1) or (2).

In one or more embodiments, the fragment is primer.

The description also provides a nucleic acid construct comprising the polynucleotide described herein.

In one or more embodiments, the nucleic acid construct is a recombinant vector or expression vector.

The description also provides a phage comprising the CTLA-4 binding molecule according to any embodiment herein.

In one or more embodiments, the CTLA-4 binding molecule is displayed on the surface of the phage.

The description also provides a host cell selected from:
(1) the host cell expressing the CTLA-4 binding molecule according to any embodiment herein;
(2) the host cell comprising a polynucleotide described herein; and/or
(3) the host cell comprising a nucleic acid construct described herein.

The description also provides a method for producing a CTLA-4 binding molecule, comprising culturing the host cells described herein under conditions suitable for producing the CTLA-4 binding molecule (such as monovalent or multivalent single domain antibodies, multispecific single domain antibodies, heavy chain antibodies, antibodies or antigen binding fragments thereof), and optionally purifying the PD-1 binding molecule from culture.

The description also provides a pharmaceutical composition, comprising the CTLA-4 binding molecule, polynucleotide, nucleic acid construct, phage or host cell according to any embodiment herein, and a pharmaceutically acceptable excipient.

In one or more embodiments, the pharmaceutical composition is used for treating cancer.

In one or more embodiments, the cancer is a CTLA-4 related cancer. Preferably, the cancer is selected from the group consisting of: lung cancer, ovarian cancer, colon cancer, rectal cancer, melanoma, carcinoma of kidney, bladder cancer, breast cancer, liver cancer, lymphoma, malignant hematological disease, head and neck cancer, glioma, gastric cancer, nasopharyngeal carcinoma, laryngeal cancer, cervical carcinoma, endometrial carcinoma, osteosarcoma, and the like.

The description also provides use of the CTLA-4 binding molecule according to any embodiment herein in the preparation of a medicament for the prevention or treatment of a cancer.

In one or more embodiments, the cancer is a CTLA-4 related cancer. Preferably, the cancer is selected from the group consisting of: lung cancer, ovarian cancer, colon cancer, rectal cancer, melanoma, carcinoma of kidney, bladder cancer, breast cancer, liver cancer, lymphoma, malignant hematological disease, head and neck cancer, glioma, gastric cancer, nasopharyngeal carcinoma, laryngeal cancer, cervical carcinoma, endometrial carcinoma, osteosarcoma, and the like.

The description also provides a method for treating or preventing a cancer, comprising administrating a patient in need thereof an effective amount of a CTLA-4 binding molecule according to any embodiment of the description, or a pharmaceutical compisiton comprising a CTLA-4 binding molecule according to any embodiment of the description.

In one or more embodiments, the cancer is a CTLA-4 related cancer. Preferably, the cancer is selected from the group consisting of: lung cancer, ovarian cancer, colon cancer, rectal cancer, melanoma, carcinoma of kidney, bladder cancer, breast cancer, liver cancer, lymphoma, malignant hematological disease, head and neck cancer, glioma, gastric cancer, nasopharyngeal carcinoma, laryngeal cancer, cervical carcinoma, endometrial carcinoma, osteosarcoma, and the like.

The description also provides a kit for detecting CTLA-4, for use in evaluating the therapeutic effect of a medicament or diagnosing cancer. The kit comprises a CTLA-4 binding molecule, polynucleotide, nucleic acid construct, phage or host cell according to any embodiment of the description.

In one or more embodiments, the kit further comprises a reagent for detecting the binding of CTLA-4 to a single domain antibody, an antibody, or an antigen binding fragment thereof. For example, the bound reagent is detected by the enzyme-linked immunosorbent assay.

In one or more embodiments, the detection reagent for binding is a detectable marker, such as biotin, that can be linked to a CTLA-4 binding molecule. The detectable marker is connected to the CTLA-4 binding molecule or present in the kit separately.

The description also provides a non diagnostic method for detecting the presence of CTLA-4 in a sample. The method comprises: incubating a CTLA-4 binding molecule according to any embodiment herein with the sample, and detecting the binding of CTLA-4 to a single domain antibody, antibody, or antigen binding fragment thereof, thereby determining the presence of CTLA-4 in the sample. The detection is an enzyme-linked immunosorbent assay.

The description also provides use of a CTLA-4 binding molecule according to any embodiment herein in the preparation of a kit for detecting CTLA-4 in a sample, evaluating the therapeutic effect of a medicament or diagnosing a cancer.

### Description of Figures

Figure 1 shows the detection of titer of Alpaca antiserum against human CTLA-4 protein.
Figure 2 shows the detection results of binding of anti human CTLA-4 antibody on 293T cells overexpressing human CTLA-4.
Figure 3A shows the detection results of anti human CTLA-4 antibody blocking the binding of B7-1 molecule on 293T cells overexpressing human CTLA-4. Figure 3B shows the detection results of anti human CTLA-4 antibody blocking the binding of B7-2 molecule on 293T cells overexpressing CTLA-4.
Figure 4 shows the effect of anti human CTLA-4 antibody on IL-2 secretion in reporter gene assay.
Figures 5A-5C show the cross binding data of humanized anti human CTLA-4 antibody to human, cynomolgus monkey and mouse CTLA-4 protein, respectively.
Figure 6 shows the binding data of humanized anti-human CTLA-4 antibody on 293T cells overexpressing human CTLA-4.
Figure 7 shows the detection results of humanized anti-human CTLA-4 antibody blocking the binding of biotin labeled B7-1 molecule on 293T cells overexpressing CTLA-4.
Figures 8A-8B show the cross reaction results of humanized anti-human CTLA-4 antibodies NB-27-z5 and NGS-12-z1 with 5300 membrane proteins, respectively.

### Detailed Description

After extensive and in-depth research and extensive screening, the inventors found a class of CTLA-4 binding molecules comprising anti-CTLA-4 single domain antibodies. The CTLA-4 binding molecule of the invention can bind to CTLA-4 with high specificity and high affinity, resulting in high biological activity, and has low immunogenicity, stable structure and good druggability. The single domain antibody of the invention is simple to generate.

### Antibody

"CTLA-4 binding molecule" as used herein is a protein that specifically binds CTLA-4, including but not limited to antibodies, antigen binding fragments of antibodies, heavy chain antibodies, nano antibodies, micro antibodies, affibodies, target binding regions of receptors, cell adhesion molecules, ligands, enzymes, cytokines, and chemokines.

Term "antibody" as used herein includes monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and FV). The term "immunoglobulin" (Ig) is used interchangeably with "antibody" herein.

The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblages. In the case of IgGs, the 4-chain unit is typically about 150,000 daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α , δ, ε, γ and µ, respectively. The γ and α classes are further divided into subclasses on the basis of relatively minor differences in the CH sequence and function, e.g., humans express the following subclasses: IgGl, IgG2A, IgG2B, IgG3, IgG4, IgA1 and IgA2.

" Heavy chain antibody" described herein is an antibody derived from camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is connected to the constant region through a hinge region like structure. Each heavy chain of camelid heavy chain antibody contains one variable region (VHH) and two constant regions (CH2 and CH3), and each heavy chain of shark heavy chain antibody contains one variable region and five constant regions (CH1-CH5). Antigen binding fragments of heavy chain antibodies include VHH and single chain heavy chain antibodies. Heavy chain antibodies may have CH2 and CH3 of human IgG Fc by fusing with the constant region of human IgG Fc.

As used herein, the terms "single domain antibody", "anti-CTLA-4 single domain antibody", "heavy chain variable region domain of heavy chain antibody", "VHH" and "nanobody" can be used interchangeably, and all refer to single domain antibodies that specifically recognize and bind to CTLA-4. Single domain antibodies are variable regions of heavy chain antibodies. Typically, single domain antibodies contain three CDRs and four FRs. Preferably, the single domain antibody of the description has CDR1 shown in SEQ ID NO: 1, CDR2 shown in SEQ ID NO: 2, and CDR3 shown in SEQ ID NO: 3. Single domain antibodies are the smallest functional antigen binding fragments. Generally, after obtaining an antibody which naturally lacks light chain and heavy chain constant region 1 (CH1), the variable region of the heavy chain of the antibody is cloned to construct a single domain antibody consisting of only one heavy chain variable region.

Binding molecules comprising two or more single domain antibodies are multivalent single domain antibodies; and binding molecules comprising two or more single domain antibodies with different specificities are multispecific single domain antibodies. Multivalent single domain antibodies or multispecific single domain antibodies are connected to multiple single domain antibodies through linkers. The linker usually consists of 1-15 amino acids selected from G and S.

The terms "heavy chain antibody" and "antibody" herein are intended to distinguish different composition forms of antibodies. Due to the similarity of their structures, the following descriptions on structures of antibodies except for light chains also apply to heavy chain antibodies.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains, namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved portions of variable domains are called the framework regions (FR). The variable domains of native heavy and light chains each comprise four FR regions (FR1, FR2, FR3, and FR4), largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domains are not involved directly in the binding of antibody to an antigen, but exhibit various effector functions, such as participation of the antibody in antibody-dependent cellular toxicity.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. In IgG, IgA and IgD antibody isotypes, the Fc region is composed of two identical protein fragments, derived from CH2 and CH3 constant domains of the antibody's two heavy chains; IgM and IgE Fc regions contain three heavy chain constant domains (CH domains 2-4) in each polypeptide chain. Although the boundaries of the Fc region of an immunoglobulin heavy chain might vary, the human IgG heavy chain Fc region is usually defined to stretch from an amino acid residue at position C226 or P230 to the carboxy-terminus of the heavy chain, wherein the numbering is according to the EU index as in Kabat. As used herein, the Fc region may be a native sequence Fc or a variant Fc.

An "antibody fragment" comprises a portion of an intact antibody, preferably the antigen binding and/or the variable region of the intact antibody. The antibody fragment is preferably an antigen binding fragment of the antibody. Examples of antibody fragments include Fab, Fab', F(ab')2 and Fv fragments; diabodies; linear antibodies; single-chain antibody molecules, scFv-Fc fragment; multispecific antibodies formed from antibody fragments; and any fragment that should be able to increase the half-life by chemical modification or by incorporation into liposomes. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. F(ab')2 antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functionfunctions of an antibody isantibodies are determined by the sequencesequences in the Fc region, the region which is also recognized by Fc receptors (FcRs) found on somecertain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method, phage-display technologies, recombinant DNA methods, and technologies for producing human or humanlike antibodies in animals that have parts or all of the human immunoglobulin loci or genes encoding human immunoglobulin sequences, single-cell sequencing methods.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable- domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies are well known in the art, such as using mice with genetically engineered immune systems. In the description, antibodies, single domain antibodies, heavy chain antibodies, etc. all include humanized variants of the antibodies.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries.

In some embodiments, the description further provides a single domain antibody, heavy chain antibody, antibody or antigen binding fragment thereof that binds to the same epitope of CTLA-4 as any anti-CTLA-4 single domain antibody of the description, that is, a single domain antibody, heavy chain antibody, antibody or antigen binding fragment thereof that can cross-compete with any single domain antibody of the description for binding to CTLA-4.

In the present invention, the CDR1 of the anti-CTLA-4 single domain antibody comprises the sequence shown in SEQ ID NO: 1,and SEQ ID NO: 1 is GX₁X₂X₃X₄X₅X₆X₇, wherein X₁ is S, L, D, F or G, X₂ is T, N or S, and X₃ is L, F or S, X₄ is D, G or E, X₅ is P, I, S, D or Y, X₆ is F, Y, H, Q or W, and X₇ is V, D, A or S. Exemplarily, CDR1 comprises the sequence shown in any one of SEQ ID NOs: 4-10.

The CDR2 of the anti-CTLA-4 single domain antibody comprises the sequence shown in SEQ ID NO: 2, SEQ ID NO: 2 is X₁X₂X₃X₄X₅X₆X₇X₈, wherein X₁ is I or M, X₂ is N, G, S or R, X₃ is R, P, S or G, X₄ is S, N, D or T, X₅ is R or G, X₆ is T, A, D, E, R, G or L, X₇ is I, F, T, S, G, R or null, and X₈ is T or null. Exemplarily, CDR2 comprises the sequence shown in any one of SEQ ID NOs: 11-18.

The CDR3 of the anti-CTLA-4 single domain antibody comprises the sequence shown in SEQ ID NO: 3, SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, G or T, X₂ is A, L, S or G, X₃ is S, G, E, V, A or K, X₄ is the P, R or L, X₅ is D, E, L, T, I or A, X₆ is K, R, V, L, S or H, X₇ is P, A, Y, F, G or Q, X₈ is F, H, T, V or S, X₉ is Y, S, L, A, H or M, X₁₀ is T, I, C, A or W, X₁₁ is D, Y, S, L or V, X₁₂ is Q, A, L, M or C, and X₁₃ is T, S, N or C, and X₁₄ is M, E, H, Q, A or null, X₁₅ is C, Y, G, H, A, N or null, X₁₆ is N, D, M, Y, S, E or null, X₁₇ is K, I, Y, R, F or null, X₁₈ is H, D, S, G or null, and X₁₉ is Y, T, D, L or null, X₂₀ is Y or null, X₂₁ is G or null, and X₂₂ is Y or null. Exemplarily, CDR3 comprises the sequence shown in any one of SEQ ID NOs: 19-26.

In one or more embodiments, the single domain antibody comprises CDR1, CDR2, and CDR3 shown in any one of the following groups a1 to a9:

**Table 1**

| Group | CDR1 | CDR2 | CDR3 |
|---|---|---|---|
| a1 | 4 | 11 | 19 |
| a2 | 4 | 12 | 20 |
| a3 | 4 | 12 | 20 |
| a4 | 5 | 13 | 21 |
| a5 | 6 | 14 | 22 |
| a6 | 7 | 15 | 23 |
| a7 | 8 | 16 | 24 |
| a8 | 9 | 17 | 25 |
| a9 | 10 | 18 | 26 |

In one or more embodiments, FR1 of the single domain antibody VHH is the FR1 region of any VHH selected from SEQ ID NOs: 27-73, FR2 of VHH is the FR2 region of any VHH selected from SEQ ID NOs: 27-73, FR3 of VHH is the FR3 region of any VHH selected from SEQ ID NOs: 27-73, and FR4 of VHH is the FR4 region of any VHH selected from SEQ ID NOs: 27-73.

In a preferred embodiment, the FR region of the single domain antibody VHH of the invention is the FR region of any VHH selected from SEQ ID NOs: 27-73. More preferably, the CDR of such antibodies is selected from any of the aforementioned groups a1 to a9. In one or more embodiments, the single domain antibody VHH is as shown in any one of SEQ ID NOs: 27-73.

The CTLA-4 binding molecule described herein may be a monovalent or multivalent single domain antibody, a multispecific single domain antibody, a heavy chain antibody, or an antigen binding fragment thereof, an antibody, or an antigen binding fragment thereof, comprising one, two, or more anti-PD-1 single domain antibodies described herein.

In some embodiments, the CTLA-4 binding molecule herein comprises an anti-CTLA-4 single domain antibody and an immunoglobulin Fc region. The Fc region suitable for the present invention can be derived from immunoglobulins of different subtypes, such as IgG (e.g., IgG1, IgG2, IgG3, or IgG4 subtypes), IgA1, IgA2, IgD, IgE, or IgM. In some embodiments, the Fc region of immunoglobulin is IgG4 Fc, and its amino acid sequence is shown in SEQ ID NO: 74.

In some embodiments, the CTLA-4 binding molecule described herein is a heavy chain antibody. The heavy chain antibody further comprises a heavy chain constant region, such as a constant region of camelid heavy chain antibody or shark heavy chain antibody. Preferably, the heavy chain constant region is shown in SEQ ID NO: 74.

The description also comprises the antibody derivatives and analogues. "Derivatives" and "analogues" refer to polypeptides that basically maintain the same biological function or activity of the antibody of the present description. The derivatives or analogues of the present description may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

Without substantially affecting the activity of the antibody, those skilled in the art may change one or more (for example, 1, 2, 3, 4, 5, 6, 7, 8, 9 or 10 or more) amino acids to the sequence of the description to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or CDR regions of the variable region. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description.

The variant forms of the antibody described herein include: homologous sequence, conservative variant, allelic variant, natural mutant, induced mutant, protein encoded by DNA that can hybridize with the coding DNA of the antibody of the description under high or low strictness conditions, and polypeptide or protein obtained by using the antiserum of the antibody of the description.

In some embodiments, the sequence of the variant of the present description may have at least 95%, 96%, 97%, 98% or 99% identity with its source sequence. The sequence identity described in the description can be measured using sequence analysis software. For example, the computer program BLAST with default parameters, especially BLASTP or TBLASTN. The description also comprises those molecules with variable regions of antibody heavy chain with CDRs, if their CDRs have more than 90% homology (preferably more than 95%, more preeferably more than 98%) with the CDRs identified here.

The antibody of the description can be prepared by conventional methods in the art, such as hybridoma technology well known in the art. The heavy chain antibody of the description can be prepared by conventional methods in the art, such as phage display technology well known in the art. Alternatively, the antibodies or heavy chain antibodies of the present description may be expressed in other cell lines. Suitable mammalian host cells can be transformed with sequences encoding the antibodies of the present description. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, Polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc. Particularly preferred cell lines are selected by determining which cell lines have high expression levels and produce antibodies with substantial CTLA-4 binding properties.

### Nucleic Acid

The description also provides polynucleotides encoding the above antibody or fragments thereof. Polynucleotides encoding heavy chain variable region, light chain variable region, heavy chain, light chain and CDRs are provided. The polynucleotide of the description can be in the form of DNAs or RNAs. DNAs include cDNAs, genomic DNAs, or synthetic DNAs. DNAs can be single stranded DNAs or double stranded DNAs. DNAs can be coding or noncoding strand DNAs.

As those skilled in the art will understand, due to the degeneracy of the genetic code, an extremely large number of nucleic acids can be prepared, all of which encode the antibody of the description or antigen binding fragment thereof. Therefore, when a specific amino acid sequence has been identified, those skilled in the art can simply modify the sequence of one or more codons without changing the amino acid sequence of the encoded protein to produce any number of different nucleic acids. Therefore, the present description also relates to polynucleotides that hybridize with the above polynucleotide sequences and have at least 50%, preferably at least 70%, more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the antibody of the description or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein.

Once the relevant sequences are obtained, they can be obtained in large quantities by recombination. They are related sequences usually cloned into vectors, transferred into cells, and then isolated from the proliferated host cells by conventional methods. The biomolecules (nucleic acids, proteins, etc.) according to the present description include biomolecules in isolated form. At present, the DNA sequence encoding the protein (or fragment thereof, or derivative thereof) of the description can be obtained completely through chemical synthesis. This DNA sequence can then be introduced into various existing DNA molecules (or such as vectors) and cells known in the art. In addition, mutations can also be introduced into the protein sequence of the description through chemical synthesis.

Therefore, the present description also relates to nucleic acid constructs, such as expression vectors and recombinant vectors, comprising the above appropriate DNA sequence and the appropriate promoter or control sequence. These vectors can be used to transform appropriate host cells to enable them to express proteins. Vectors usually contain sequences for plasmid maintenance and for cloning and expressing exogenous nucleotide sequences. The sequences (collectively referred to as "flanking sequence" in some embodiments) generally comprises one or more of the following nucleotide sequences: promoter, one or more enhancer sequences, replication origin, transcription termination sequence, complete intronic sequence comprising donor and receptor splice sites, sequence encoding leader sequence for polypeptide secretion, ribosome binding site, polyadenylation sequence, a multi-linker region for inserting nucleic acids encoding antibodies to be expressed and an optional marker element.

Host cells can be prokaryotic cells, such as bacterial cells; or lower eukaryotic cells, such as yeast cells; or higher eukaryotic cells, such as mammalian cells. Representative examples are: Escherichia coli, Streptomyces; bacterial cells of Salmonella typhimurium; fungal cells such as yeast; insect cells of Drosophila S2 or Sf9; animal cells of CHO, COS7, 293 cells, etc.

Transformation of host cells with recombinant DNA can be performed using conventional techniques known to those skilled in the art. When the host is a prokaryote such as Escherichia coli, competent cells that can absorb DNA can be harvested after the exponential growth period and treated with CaCh method, the steps of which are well known in the art. Another method involves the use of MgCh. If necessary, the transformation can also be performed by electroporation. When the host is eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the description. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

### Use of Treatment and Pharmaceutical Composition

By constructing a nanobody library, the inventor discovered, expressed and purified a number of nanobodies that can bind to CTLA-4 protein. All aspects of the antibodies described herein can be used to prepare drugs to prevent or treat various conditions and diseases described herein, especially those related to cells expressing CTLA-4. In some embodiments, the conditions and diseases are cancers, including but not limited to lung cancer, ovarian cancer, colon cancer, rectal cancer, melanoma, carcinoma of kidney, bladder cancer, breast cancer, liver cancer, lymphoma, malignant hematological disease, head and neck cancer, glioma, gastric cancer, nasopharyngeal carcinoma, laryngeal cancer, cervical carcinoma, endometrial carcinoma, osteosarcoma, and the like.

The pharmaceutical composition herein comprises the binding molecules described herein, as well as pharmaceutically acceptable excipients, including but not limited to diluents, vehicles, solubilizers, emulsifiers, preservatives, and/or adjuvants. The excipients are preferably non-toxic to the recipient at the dose and concentration used. Such excipients include (but are not limited to): saline, buffer, glucose, water, glycerol, ethanol, and their combinations. In some embodiments, the pharmaceutical composition may contain substances for improving, maintaining, or retaining, for example, the pH, permeability, viscosity, clarity, color, isotonicity, odor, sterility, stability, dissolution or release rate, absorption, or permeation of the composition. These substances are known in the prior art. The optimal pharmaceutical composition can be determined according to the expected route of administration, mode of delivery, and required dose.

Pharmaceutical compositions for in vivo administration are usually provided in the form of sterile formulations. Sterilization is achieved by filtration through a sterile filter membrane. When the composition is lyophilized, this method can be used for sterilization before or after lyophilization and rehydration. The pharmaceutical composition of the present description may be selected for parenteral delivery. Compositions for parenteral administration may be in lyophilized form or stored in solution. For example, it is prepared by conventional methods with normal saline or aqueous solution comprising glucose and other adjuvants. Parenteral compositions are usually placed in containers with sterile access holes, such as intravenous solution strips or vials with plugs that can be pierced by subcutaneous injection needles. Alternatively, compositions may be selected for inhalation or delivery through the digestive tract, such as oral. The preparation of the pharmaceutically acceptable composition is within the art. Other pharmaceutical compositions will be apparent to those skilled in the art, including formulations comprising antibodies in sustained or controlled release delivery formulations. The techniques used to prepare a variety of other sustained or controllable delivery modes (such as liposome carriers, bioerodible particles or porous beads, and deposit injection) are also known to those skilled in the art.

Once the pharmaceutical composition is prepared, it is stored in sterile vials in the form of solution, suspension, gel, emulsion, solid, crystal or dehydrated or lyophilized powder. The formulation may be stored in ready to use form or rehydrated before administration (e.g., lyophilized). The description also provides a kit for generating a single dose administration unit. The kit of the description can each contain a first container with dried protein and a second container with aqueous formulation. In some embodiments of the present description, a kit comprising single-chamber and multi-chamber pre-filled syringes (e.g., liquid syringes and lyophilized syringes) are provided.

The present description also provides a method for treating a patient (especially a patient's CTLA-4 related disease) by administering a binding molecule or a pharmaceutical composition thereof according to any embodiment of the present description. Terms "patient", "subject", "individual" and "object" are used interchangeably herein, including any organism, preferably animals, more preferably mammals (such as rats, mice, dogs, cats, rabbits, etc.), and most preferably humans. "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a redeuced rate of tumor metastasis or tumor growth). The treatment scheme for effectively treating patients can vary according to many factors, such as the patient's disease status, age, weight, and the ability of the therapy to stimulate the subject's anti-cancer response.

The therapeutically effective amount of the pharmaceutical composition comprising the binding molecule of the present description to be used will depend on, for example, the degree and the target of treatment. Those skilled in the art will understand that the appropriate dose level for treatment will vary in part depending on the molecule delivered, the indication, the route of administration, and the size (body weight, body surface or organ size) and/or condition (age and general health condition) of the patient. In some embodiments, clinicians may titrate the dose and change the route of administration to obtain the optimal therapeutic effect. For example, about 10 micrograms/kg body weight per day to about 50 mg/kg body weight.

The dosing frequency will depend on the pharmacokinetic parameters of the bound molecules in the formulation used. Clinicians typically administer the compositions until a dosage that achieves the desired effect. The composition may therefore be administered as a single dose, or over time as two or more doses (which may or may not contain the same amount of the desired molecule), or as a continuous infusion through an implanted device or catheter.

The route of administration of the pharmaceutical composition is according to known methods, such as oral, intravenous, intraperitoneal, intracerebral (intraparenchymal), intraventricular, intramuscular, intraocular, intra-arterial, portal vein or intralesional injection; by continuous release system or by implantable device.

### Diagnosis, Detection, and Kit

The binding molecule of the present description can be used in assays due to its high avidity with CTLA-4, such as binding assays to detect and/or quantify CTLA-4 expressed in tissues or cells. Binding molecules such as single domain antibodies can be used in further studies investigating the function of CTLA-4 in disease. The methods for detecting CTLA-4 are roughly as follows: obtaining cell and/or tissue samples; and detecting the level of CTLA-4 in the sample.

The CTLA-4 binding molecule of the description can be used for diagnostic purposes to detect, diagnose, or monitor CTLA-4 related diseases and/or conditions. The description provides the detection of the presence of CTLA-4 in a sample using a classical immunohistological method known to those skilled in the art. Detection of CTLA-4 can be performed *in vivo* or *in vitro.* Examples of methods suitable for detecting the presence of CTLA-4 include ELISA, FACS, RIA, and the like.

For diagnostic uses, binding molecules such as single domain antibodies are usually labeled with detectable labeling groups. Suitable labeling groups include (but are not limited to): radioisotopes or radionuclides (e.g., 3H, 14C, 15N, 35S, 90Y, 99Tc, 111In, 125I, 131I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present description.

Another aspect of the present description provides a method for detecting the presence of a test molecule that competes with an antibody of the present description to bind CTLA-4. An example of one such assay would involve detecting the amount of free antibody in a solution comprising an amount of CTLA-4 in the presence or absence of the test molecule. An increase in the amount of free antibody (i.e., antibody that does not bind CTLA-4) will indicate that the test molecule can compete with the antibody for binding to CTLA-4. In one embodiment, the antibody is labeled with a labeling group. Alternatively, the test molecule is labeled, and the amount of free test molecule is monitored in the presence or absence of the antibody.

The description also provides a detection kit for detecting CTLA-4 level. The kit comprises an antibody that recognizes CTLA-4 protein, a lysis medium for dissolving samples, and general reagents and buffers required for detection, such as various buffers, detection markers, detection substrates, etc. The detection kit can be an in vitro diagnostic device.

The present invention will be described below in the form of specific examples. It should be understood that these examples are merely illustrative and are not intended to limit the scope of the present description. Unless otherwise specified, the methods and materials used in the examples are conventional materials and methods in the art.

### Example

### Example 1. Immunization of Alpaca

### 1.1 Preparation of Immunogen:

The sequence of CTLA-4 protein was obtained from NCBI, and was fused with the sequence of human IgG Fc fragment, and the eukaryotic expression vector of pCDNA3.4 (Thermo) plasmid was synthesized and constructed by Nanjing Genscript Company. The synthesized plasmid was expressed using ExpiCHO^{™} (Thermo Fisher) expression system. After expression, 5 ml of protein A pre-packed column (GE) was used for one-step affinity purification, and the purified sample was replaced into PBS buffer. After the purity was identified by SDS-PAGE electrophoresis gel and HPLC, and the activity was identified by ELISA, the sample was split and frozen in -80°C refrigerator for subsequent immunization.

### 1.2 Immunization of Alpaca:

For first immunization, 1000 µg of antigen (CTLA-4.hFc) was mixed with the adjuvant (GERBU FAMA). An alpaca was immunized subcutaneously at four sites on the back with an immune amount of 1 mL at each site. The second immunization was performed at an interval of 3 weeks. The second to the ninth immunization: the amount of immune antigen was 500 µg, and the alpaca was immunized subcutaneously at four sites on the back with an immune amount of 1 mL at each site, the interval of each immunization being one week.

### 1.3 Detection of Immune Serum Titer:

### 1.3.1 Detection of Titer at Protein Level

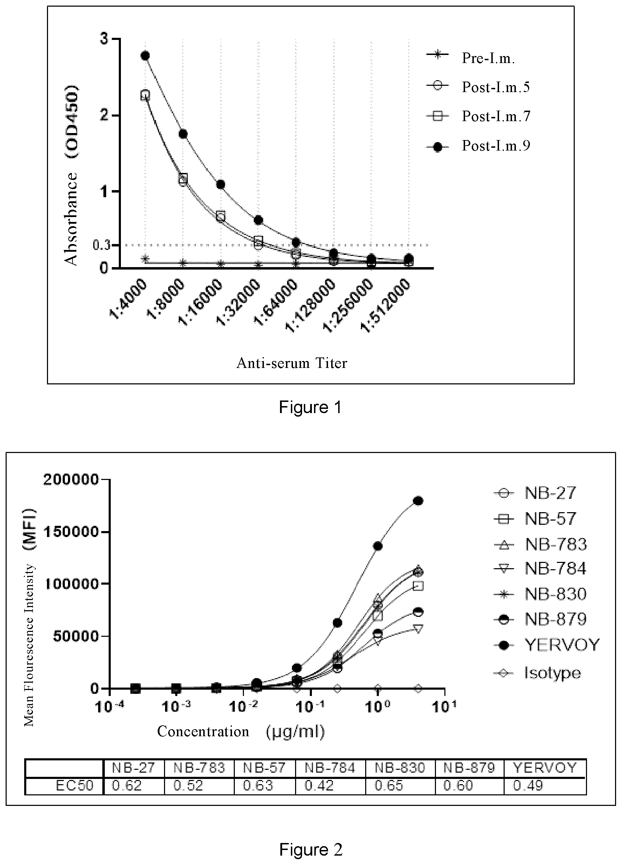

CTLA-4.His antigen (Acrobiosystems) was coated overnight at 4 degrees. After blocking and washing, the gradient diluted serum was added to the ELISA plate for incubation, and then incubated with the anti-llama IgG HRP (Abcam) antibody. After washing, TMB chromogenic solution was added for development, and the reaction was terminated with 2 M HCl. Then the absorbance value at OD450 nm was detected with a microplate reader. As shown in Figure 1, the titer of Alpaca reached a high level (>64000) after 9 immunizations.

### Example 2. Construction and Screening of Nanobody Immune Library for CTLA-4

(1) After nine immunizations, 100 mL of camelid peripheral blood lymphocytes were extracted, and total RNA was extracted. RNA was extracted according to the instructions of RNAiso reagent of Takara.
(2) The first strand of cDNA was synthesized with RNA as template and oligo dT as primer according to the instructions of reverse transcriptase of Takara.
(3) The variable region coding gene of heavy chain antibody was obtained by nested PCR using PrimeSTAR high fidelity DNA polymerase. The variable region fragment of the heavy chain antibody was amplified by nested PCR:
   First round of PCR:
      Upstream primer: GTCCTGGCTGCTCTTCTACAAGGC ( SEQ ID NO:77)
      Downstream primer: GGTACGTGCTGTTGAACTGTTCC (SEQ ID NO:78)
         The fragment between the heavy chain antibody guide peptide and antibody CH2 was amplified, annealed at 55°C for 30 cycles; a DNA fragment of about 600 bp was recovered as a template for the second round of PCR.
   Second round of PCR:
      Upstream primer: GATGTGCAGCTGCAGGAGTCTGGRGGAGG (SEQ ID NO:79)
      Downstream primer: GGACTAGTGCGGCCGCTGGAGACGGTGACCTGGGT (SEQ ID NO:80)
         The fragment (long fragment and short fragment) between the FR1 region and the long and short hinge regions of the heavy chain antibody was amplified, annealed at 55°C for 30 cycles, and the target fragment was recovered.The result showed that the size of the fragment was about 500 bp, that is, the nanobody gene electrophoresis band was about 500 bp.
(4) The phage pME207 and PCR amplification products were digested with Sfi I and Not I (NEB), respectively. After recovery and quantification, the two fragments were ligated with T4 DNA ligase (Takara) at a molar ratio of 1:3 and ligated overnight at 16°C.
(5) After ethanol precipitation, the ligated product was dissolved in 100 µL sterile water and electroporated into Escherichia coli TG1 in ten times. 100µL of the bacterial solution was taken after electric shock and culture, diluted by multiple ratio, coated on an ampicillin LB culture plate, the storage capacity was calculated, and the rest was coated with ampicillin 2×YT culture plate, at 37°C, invertly cultured for 13-16 h. After scraping and washing the colonies on the culture plate with 10 ml, 2×YT medium, 25% glycerol at the final concentration was added, split, and stored at -80°C for further use. The size of the storage capacity is 4.3 × 10⁹. To detect the insertion rate of the library, 48 clones were randomly selected for colony PCR, and the results showed that the insertion rate had reached more than 90%.
(6) According to the calculated library capacity results, viable cells with 10 times the library capacity were seeded in 200ml of 2×YT (comprising 2% glucose, 100 µg/ml ampicillin), cultured at 37°C for 200 r/min until the OD600 reached 0.5, auxiliary phage was added according to the multiplicity of infection of 20:1, and left for 30 min at 37°C, 200 r/min for 30 min. The culture was centrifuged, and the pellet was resuspended with 200 ml of 2×YT (comprising 100 µg/ml ampicillin and 50 µg/ml kanamycin), incubated overnight at 37°C, 250 r/min, centrifuged at 8000rpm to obtain the supernatant, added with 5×PEG/NaCl solution, placed on ice for 60 min, centrifuged at 8000rpm for 30 min. The pellet was resuspended in 5 ml of PBS to obtain the single domain heavy chain antibody (VHH) immune library against CTLA-4, and 10 µL was taken to determine the titer, and the rest were split at -80°C for storage.
(7) CTLA-4 was coated on an ELISA plate at 5 µg/ml, 100 µl per well, and placed overnight at 4 °C. At the same time, a negative control was set up. The next day, 200 µL, 3%BSA were added to the five wells and blocked for 2 hours at room temperature. Two hours later, they were washed three times with PBST (comprising 0.05% Tween 20 in PBS). After the plate was washed, 100 µL of phage pre-blocked with 5% skim milk (2-3 × 10¹¹ tfu immunized camelid nanobody phage display gene library) was added, left for 1.5 hours at room temperature, and then the supernatant after negative screening was transferred to the target antigen coated well for 1.5 hours at room temperature. It was washed with PBST (comprising 0.05% Tween 20 in PBS) for 12 times to wash out the unbound phage. The phage specifically bound to CTLA-4 was dissociated with Glycine (SIGMA), and the eluted phage was neutralized by Tris (Invitrogen, 1 M, pH 8.0) and infected with TG1 in logarithmic phase. After propagation and expansion, the next round of "adsorption-elution" was carried out. Finally, the eluted phage was impregnated with TG1, and the TG1 was induced by IPTG (Thermo) to express nanobody. The supernatant of TG1 expression was taken for ELISA binding detection and blocking activity detection. The secondary antibodies used for ELISA binding detection and blocking of binding to Biotinylated Human B7-1/CD80 Protein, Fc molecule (Acrobiosystem) were anti-c-myc antibody HRP (Bethyl), and HRP labeled streptavidin (Thermo), respectively. In addition, the supernatant of TG1 expression was used for detection of FACS binding on CTLA-4 cells overexpressing 293T. The primary antibody was biotin-anti-his antibody (GenScript), and the fluorescent antibody was PE streptavidin (Biolegend).
(8) After sequence analysis, combined with ELISA and FACS detection results, 9 nanobodies with blocking activity were selected as candidate antibodies.

### Example 3. Expression and Purification of Candidate Antibodies

The nanobody was constructed on pCDNA3.4-IgG4 vector to form VHH-IgG4, and then expressed by ExpiCHO^{™} (Thermo Fisher) expression system. After one week of expression, the supernatant was collected for protein A (GE) purification. Then Nanodrop was used to detect the protein quality, and HPLC was used to detect the protein purity. The purity and yield of the obtained protein met the requirment of subsequent tests.

### Example 4. Characterization of Candidate Antibodies

### (1) Detection of Affinity at Protein Level

The binding kinetics and affinity of antibody to human CTLA-4 his tag antigen were determined using surface plasmon resonance (SPR). The purified antibody was flowed through the sensor chips pre-immobilized with protein A, and the antibody was captured by protein A. Then five different concentrations of CTLA-4.his protein were used as the mobile phase, and the binding time and dissociation time were 30min and 60min, respectively. The binding rate (ka), dissociation rate (kd), and equilibrium constant (kd) were analyzed using Biacore Evaluation Software 2.0 (GE). The marketed CTLA-4 antibody medicaments Yevory were selected as controls. BMK1 is an IgG4 antibody prepared according to the sequence of Yevory from https://www.drugbank.ca/, BMK2 is an IgG4 antibody prepared according to the sequence in the patent CN 107400166 A of Suzhou Alphamab Co., Ltd.. The amino acid sequences of each candidate antibody, BMK1, and BMK2 are shown in Table 5, and the CDR sequences of each candidate antibody are shown in Table 6. The protein level affinity detection results are shown in Table 1.

**Table 1. Affinity Detection Results of Different Antibodies**

| Sample | SPR | | |
|---|---|---|---|
| | ka (1/Ms) | kd (1/s) | KD (M) |
| NB-27 | 9.45E+04 | 1.90E-04 | 2.01E-09 |
| NB-57 | 1.30E+05 | 1.27E-04 | 9.75E-10 |
| NB-783 | 1.01E+05 | 7.50E-05 | 7.44E-10 |
| NB-784 | 3.20E+04 | 2.04E-04 | 6.37E-09 |
| NB-879 | 8.09E+04 | 1.68E-04 | 2.08E-09 |
| NGS-12 | 1.90E+05 | 2.50E-04 | 1.32E-09 |
| NGS-16 | 8.80E+05 | 3.70E-04 | 4.20E-10 |
| BMK1 | 7.83E+04 | 9.56E-04 | 1.22E-08 |
| BMK2 | 2.62E+04 | 3.41E-05 | 1.30E-09 |
| Yervoy | 8.01E+04 | 5.81E-04 | 7.25E-09 |

### (2) Ligand Competition Assay at Protein Level

ELISA was used to identify whether the VHH-IgG4 antibody competed with the ligand biotinylated human B7-1/CD80 protein, Fc protein (Acrobiosystem) for binding to human CTLA-4.hFc extracellular protein. Briefly, CTLA-4.his antigen was diluted with PBS to 1.5 µg/mL, and each well of an ELISA plate was filled with 100 µL antigen and coated overnight at 4 °C. The next day, the plates were washed twice with PBST (added with 0.5% TWEEN-20), then blocked with 3 %BSA at 37 °C for 1.5 h, and then washed twice with PBST. Biotinylated human B7-1/CD80 protein, Fc protein was diluted to 5.2 µg/mL. The VHH-IgG4 antibody was diluted to 2000 nM, and then 4-fold diluted for 8 gradients. To each well, 50 µL biotinylated human B7-1 / CD80 protein, Fc protein and 50 µL diluted antibody were added, and the ELISA plate was incubated at 37 °C for 1 h. After washing three times, 100 µL HRP-SA antibody (Biolegend) diluted at 1:2000 was added into each well and incubated for 30 min. Then the plates were washed three times with PBST, and 100 µL TMB chromogenic solution was added into each well and reacted for 3 to 5 min. Then, 100 µL of 3N hydrochloric acid was added into each well to stop the reaction. After the reaction was terminated, OD450/650 was selected on the Tecan microplate reader for detection. Graphprism 8.0 software was used to make dose-response curves and calculate IC50. The results showed that all nine candidate antibodies, as well as BMK and Yervoy, exhibited ligand competitive activity, except isotype, whose amino acid sequence was shown in SEQ ID NO:2 in CN 106146653 A. The results are summarized in Table 2.

### (3) Binding Assay of Homologous Proteins

An ELISA plate was coated with CTLA-4 and its homologous protein PD-1. his tag protein (ACRO Biosystems), ICOS. His tag (ACRO Biosystems) , CD28. His tag (ACRO Biosystems) at a concentration of 1 µg/ml and placed overnight at 4 °C. After blocking and washing, the diluted antibodies were added to the ELISA plate and incubated for 2 hours at room temperature. After washing, a secondary antibody, Goat anti-Human IgG-Fc Fragment Antibody HRP (Bethyl) , was added and incubated for 1 h at room temperature. After washing, TMB chromogenic solution was added, and then 3N HCl was added to stop the reaction. A microplate reader was used to read the OD450 value. The results showed that the nine candidate antibodies, as well as Yervory and BMK only bound to CTLA-4 protein, and had no cross reaction with CTLA-4 homologous proteins (PD-1, ICOS, CD28). The results of the nine candidate antibodies are summarized in Table 2.

### (4) Cross-Species Reactivity Assay

An ELISA plate was coated with CTLA-4. his tag protein (ACRO Biosystems) of different species (human, cynomolgus monkey, and murine) at a concentration of 1 µg/ml, and placed at 4 °C overnight. After blocking and washing, the diluted antibodies were added to the ELISA plate and incubated at room temperature for 2 hours. After washing, secondary antibody Goat anti-Human IgG-Fc Fragment Antibody HRP (Bethyl) was added and incubated at room temperature for 1 hour. After washing, TMB chromogenic solution was added, and then the reaction was terminated with 2M HCl, and the OD450 value was read using a microplate reader. The results showed that all nine candidate antibodies and BMK1 bound to human and cynomolgus monkey CTLA-4, and didn't bind to mouse CTLA-4. The results are shown in Table 2.

**Table 2. Summary of ELISA Results of Anti-CTLA-4 Antibodies**

| Sample | Binding to Homologous Proteins | | | Cross-Species Reactivity | | IC50 of Blocking Assay (nM) |
|---|---|---|---|---|---|---|
| | PD-1 | CD28 | ICOS | mouse | Cynomolgus monkey | B7-1 |
| NB-27 | - | - | - | - | ++ | 39.70 |
| NB-57 | - | - | - | - | ++ | 37.20 |
| NB-783 | - | - | - | - | ++ | 36.60 |
| NB-784 | - | - | - | - | ++ | - |
| NB-879 | - | - | - | - | ++ | 55.85 |
| NGS-08 | - | - | - | - | ++ | 61.7 |
| NGS-12 | - | - | - | - | ++ | 0.57 |
| NGS-13 | - | - | - | - | ++ | 34.4 |
| NGS-16 | - | - | - | - | ++ | 2.28 |
| BMK1 | - | - | - | -/+ | ++ | 9.44 |
| BMK2 | - | - | - | - | ++ | 8.26 |
| Yervoy | - | - | - | -/+ | ++ | 10.42 |
| Note: "-" means OD450<0.3, no binding; "+" means 0.3≤OD450≤1, "+ +" means OD450>1. | | | | | | |

### (5) Binding activity assays at the cellular level

HEK293T cells expressing CTLA-4 extracellular domain were plated in 96-well plates with 3×10⁵ cells per well, and then the gradient diluted candidate antibodies were incubated with the 293T cells overexpressing CTLA-4 at 2 to 8 °C for half an hour. After washing, detection antibody anti-human IgG PE (Jackson Immuno Research, Code: 109-117-008) was added for incubation, and then CytoFLEX flow cytometry was used for detection. The EC50 (nM) of antibody was calculated by curve fitting with Graphprism software. Isotype is a negative control, whose sequence is shown in SEQ ID NO:2 of CN 106146653A). The results of some candidate antibodies are shown in Figure 2. The EC50 of the other candidate antibodies was 0.4 to 0.7 nM.

### (6) Detection of Ligand Competitive Activity at Cellular Level

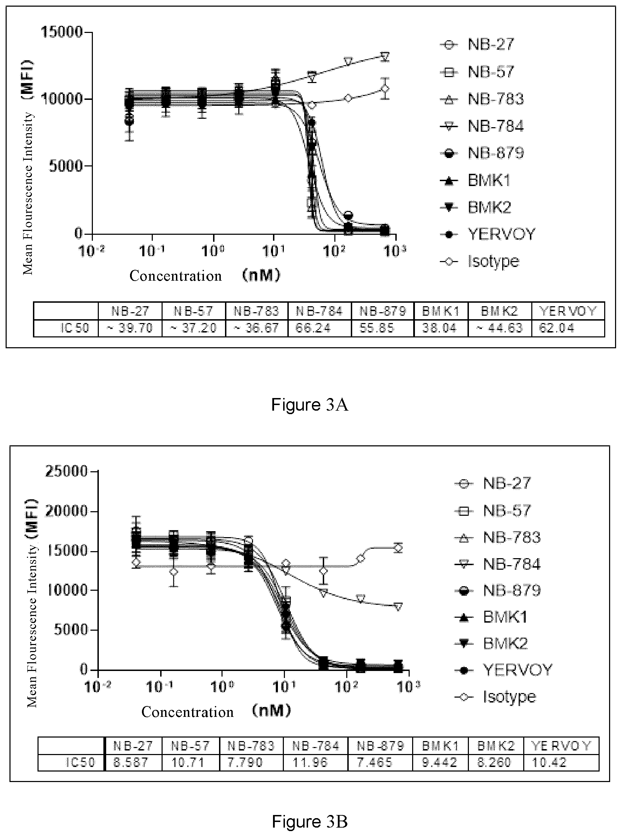

The ligands Biotinylated Human B7-1 / CD80 Protein, Fc protein (Acrobiosystem), and Biotinylated Human B7-2 / CD80 Protein, Fc protein (Acrobiosystem) were diluted to a fixed concentration, respectively. HEK293T cells expressing the extracellular domain of CTLA-4 were plated in 96-well plates with 3×10⁵ cells per well, to which 50 µL gradient diluted antibody and 50 µL ligand were added and incubated at 4 °C for 30 min. After washing, PE streptivin (Biolegend), a detection reagent, was added and incubated for another 30 min. After incubation, the plates were washed, resuspended and detected by CytoFLEX flow cytometry. The IC50 of antibody was calculated by curve fitting with Graphprism software. The results showed that all candidate antibodies except NB-784 antibody could compete with the ligands for binding to CTLA-4, and the results of some candidate antibodies were shown in Figure 3A and Figure 3B.

### Example 5: Antibody Functional Activity Assay (Reporter Gene Assay)

A test buffer (RMPI1640+10%FBS buffer) was used to adjust the density of GS-J1/CD28 cells (GenScript) to 2×10⁶ cells /ml. The density of GS-C1/CD80 cells (GenScript) target cells was adjusted to 1×10⁶ cells /ml with antibiotic-free medium. The antibody to be tested was gradiently diluted with test buffer, and CTLA-4 protein was diluted to 100 µg/mL with test buffer. Into each well of a 384-well plate, 20 µL GS-J1/CD28 cells, 20µL GS-C1/CD80 cells, 20µL CTLA-4 protein, and 20µL of antibody or test buffer were added successively, and the 384-well plate was placed in a cell incubator for 24 hours. After 24 hours, the plate was centrifuged and the cell supernatant was taken for quantification of IL-2 concentration with an IL-2 quantitative detection kit (Cisbio). The detection results were subjected to curve fitting by Graphprism software, and the EC50 (nM) of antibody was calculated.

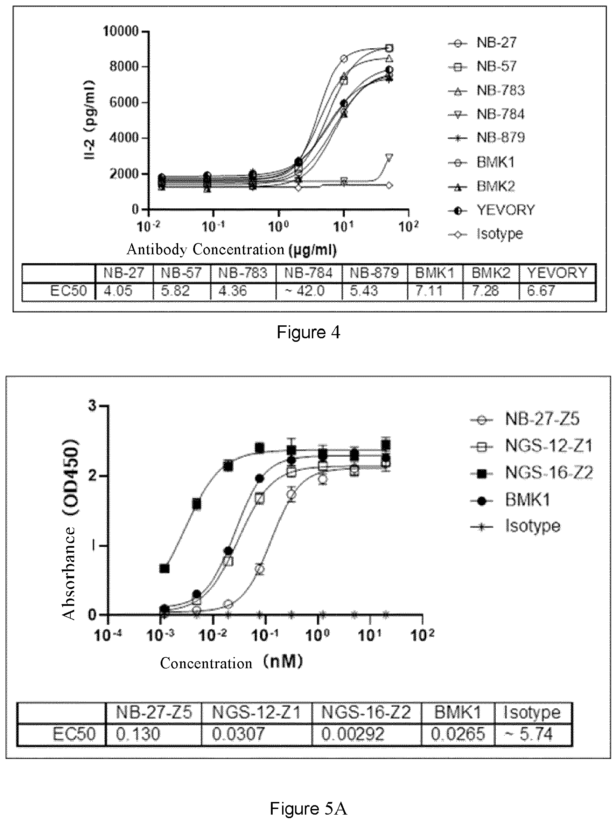

As shown in Figure 4, each candidate antibody has good functional activity in the functional detection method of reporter gene test. The EC50 of NB-27, NB-57, NB-783 and NB-879 is between 4 and 6 nM, and that of BMK1, BMK2 and Yervory is about 7nM. NB-784 has poor functional activity.

### Example 6: Humanized Modification and Affinity Detection of VHH Antibody

Referring to the method of humanizing nanobodies (J. Biol. Chem. 2009; 284: 3273-3284), four VHH antibodies, NB-27, NGS-12, NGS-16, and NB-879 were humanized using the method of CDR region transplantation. The modified antibody sequences of each antibody are shown in Table 5. In IgBLAST (http://www.ncbi.nlm.nih.gov/igblast/), germline with high homology with NB-27, NGS-12, NGS-16, NB-879 was selected as the template in the database. Biacore was used to detect the affinity of the antibody before and after humanization. The purified antibody was flowed through the sensor chip pre-fixed with protein A, and the antibody was captured by protein A. Then five different concentrations of CTLA-4.His protein were used as the mobile phase, and the association time and dissociation time were 30 min and 60 min, respectively. The binding rate (ka), dissociation rate (kd), and equilibrium constant (kd) were analyzed using Biacore Evaluation Software 2.0 (GE). The detection results are shown in Table 3. The affinity of each modified antibody did not decrease, and even increased.

**Table 3. Affinity (SPR) Detection Results of Humanized Antibody**

| Sample | Humanization Degree (%) | Affinity (SPR detection) | | |
|---|---|---|---|---|
| | | ka (1/Ms) | kd (1/s) | KD (M) |
| NGS-12 | 76.00% | 1.90E+05 | 2.50E-04 | 1.32E-09 |
| NGS-12-z1 | 83.30% | 5.40E+05 | 7.50E-04 | 1.39E-09 |
| NGS-12-z2 | 82.30% | 1.50E+05 | 2.70E-04 | 1.80E-09 |
| NGS-12-z3 | 82.30% | 1.90E+05 | 3.20E-04 | 1.68E-09 |
| NGS-12-z4 | 81.20% | 2.00E+05 | 3.30E-04 | 1.65E-09 |
| NGS-12-z5 | 80.20% | 2.50E+05 | 2.90E-04 | 1.16E-09 |
| NGS-16 | 71.90% | 8.80E+05 | 3.70E-04 | 4.20E-10 |
| NGS-16-z1 | 81.20% | 1.26E+06 | 4.73E-03 | 3.75E-09 |
| NGS-16-z2 | 80.20% | 6.80E+05 | 3.80E-04 | 5.59E-10 |
| NGS-16-z3 | 80.20% | 1.08E+06 | 3.05E-03 | 2.82E-09 |
| NGS-16-z4 | 80.20% | 5.90E+05 | 3.60E-04 | 6.10E-10 |
| NGS-16-z5 | 79.20% | 5.80E+05 | 2.70E-04 | 4.66E-10 |
| NGS-16-z6 | 78.10% | 4.20E+05 | 2.00E-04 | 4.76E-10 |
| NGS-16-z7 | 80.20% | 1.39E+06 | 6.76E-03 | 4.86E-09 |
| NGS-16-z8 | 79.20% | 1.07E+06 | 5.70E-04 | 5.33E-10 |
| NGS-16-z9 | 79.20% | 1.55E+06 | 6.17E-03 | 3.98E-09 |
| NGS-16-z10 | 78.10% | 1.08E+06 | 6.50E-04 | 6.02E-10 |
| NGS-16-z11 | 77.10% | 1.05E+06 | 4.50E-04 | 4.29E-10 |
| NB-27 | 73.1% | 2.62E+05 | 3.32E-04 | 1.27E-09 |
| NB-27-z1 | 79.4% | 3.99E+05 | 4.55E-04 | 1.14E-09 |
| NB-27-z2 | 78.4% | 3.66E+05 | 3.45E-04 | 9.43E-10 |
| NB-27-z3 | 78.4% | 3.41E+05 | 3.81E-04 | 1.12E-09 |
| NB-27-z5 | 77.3% | 2.58E+05 | 3.12E-04 | 1.21E-09 |
| NB-27-z6 | 77.3% | 2.59E+05 | 3.39E-04 | 1.31E-09 |
| NB-27-z7 | 76.3% | 2.61E+05 | 3.05E-04 | 1.17E-09 |
| NB-27-z10 | 80.4% | 2.65E+05 | 3.76E-04 | 1.42E-09 |
| NB-879 | 69.00% | 9.27E+04 | 1.33E-03 | 1.44E-08 |
| NB-879-z1 | 78.40% | 1.72E+05 | 3.02E-03 | 1.76E-08 |
| NB-879-z2 | 77.30% | 1.71E+05 | 3.07E-03 | 1.80E-08 |
| NB-879-z3 | 77.30% | 1.74E+05 | 3.17E-03 | 1.82E-08 |
| NB-879-z4 | 77.30% | 2.02E+05 | 3.45E-03 | 1.71E-08 |
| NB-879-z5 | 76.30% | 1.88E+05 | 3.18E-03 | 1.69E-08 |
| NB-879-z6 | 76.30% | 1.60E+05 | 3.30E-03 | 2.07E-08 |
| NB-879-z7 | 75.30% | 1.28E+05 | 2.46E-03 | 1.92E-08 |
| NB-879-z11 | 78.40% | 2.42E+05 | 8.24E-03 | 3.40E-08 |
| NB-879-z12 | 77.30% | 2.67E+05 | 9.49E-03 | 3.56E-08 |
| NB-879-z13 | 77.30% | 4.30E+04 | 2.23E-03 | 5.19E-08 |
| NB-879-z14 | 75.30% | 1.08E+05 | 1.88E-03 | 1.73E-08 |
| NB-879-z15 | 75.30% | 1.17E+05 | 2.16E-03 | 1.84E-08 |

### Example 7. Characterization of Humanized VHH Antibodies

### (1) Cross-Species Reactivity Assay

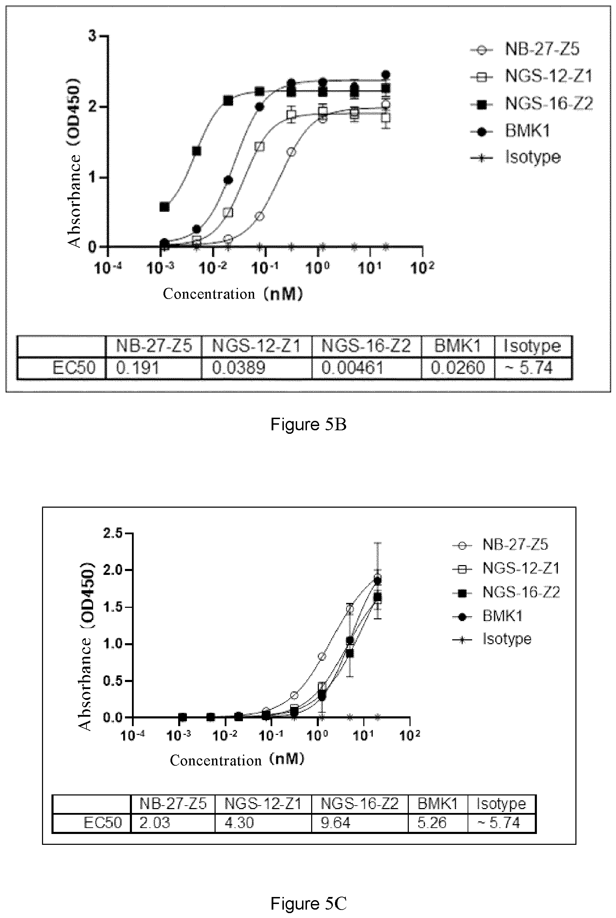

The binding of three humanized VHH antibodies (NB-27-Z5, NGS-12-Z1, NGS-16-Z2) to CTLA-4 protein of human, cynomolgus monkey, and mouse was detected by ELISA. The detection method was shown in the cross-species reactivtiy assay in Example 4, and the results are shown in Figures 5A-5C. All three humanized antibodies have good binding with the CTLA-4 protein of human, cynomolgus monkey, and mouse.

### (2) Detection of Binding Activity at Cellular Level

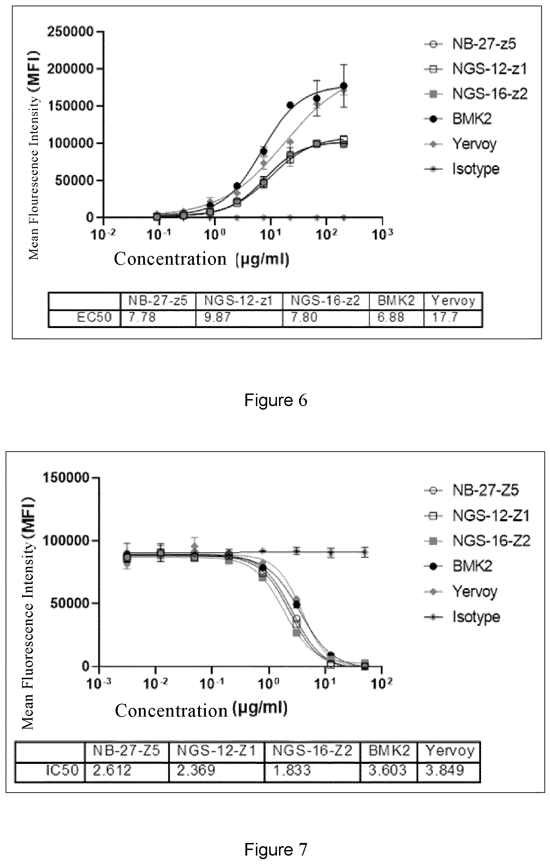

The binding activity of humanized VHH antibodies to 293T cells overexpressing CTLA-4 was detected by FACS, as shown in the cell level binding activity detection section of Example 4. As shown in Figure 6, the binding EC50 of the three modified antibodies to 293T cells overexpressing CTLA-4 ranged from 7 to 10 nM.

### (3) Experiments on Ligand Competition at Cellular Level

The humanized VHH antibodies competing with B7-1 protein for binding to 293T cells overexpressing CTLA-4 was detected by FACS, as shown in the cell level ligand competition activity detection section of Example 4. As shown in Figure 7, the IC50 values of three VHH antibodies, NGS-12-Z1, NGS-16-Z2, and NB-27-Z5, were 2.6 nM, 2.4 nM, and 1.8 nM, respectively, showing that the modifications all maintained good ligand competitive activity.

### Example 8. Verification of Specificity of Humanized VHH antibody

### 1) Membrane Protein Cross-Reactivity

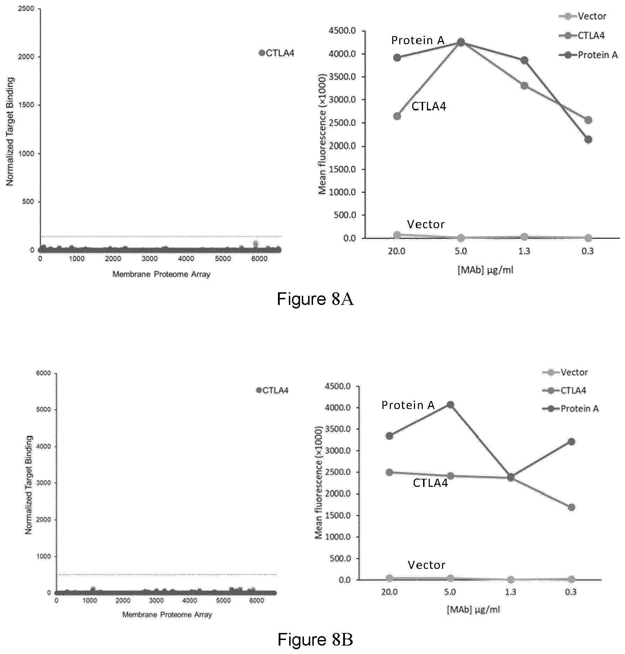

Membrane protein cross-reaction was performed using the membrane protein array screening platform (Membrane Proteome Array, MPA) developed by Integral molecular, United States. 5300 different human membrane proteins were displayed on the cell surface by transfecting HEK293 cells, and the binding signals of the antibodies on these proteins were detected by FACS to evaluate the specificity of the antibodies to be detected. The results of MPA screening (Fig. 8A and Fig. 8B) showed that NB-27-Z5 and NGS-12-Z1 antibodies only bound to human CTLA-4 protein and did not bind to more than 5000 other proteins.

### 2) Tissue Cross-Reactivity

34 tissues were selected for frozen section, dried at room temperature, and fixed with acetone. Blocking was performed using Reagent A and Reagent B of IHC Biotin Block Kit (Sangon, E674001). Biotin labeled antibody samples were incubated for 30min, and horseradish peroxidase labeled streptavidin (Abcam, ab7403) was added for incubation for 15min after washing. DAB development, hematoxylin counterstaining, neutral plastic sealing, natural air drying, and then microscopic examination were performed. The positive controls were anti-CTLA-4 antibodies (BMK1 and BMK2), and the negative control was the IgG4 isotype.

The results are shown in Table 4. BMK1 and BMK2 have a small amount of positive reactivity (less than 5%) in few tissues (testis and tonsil); NGS-16-Z2 has a small amount of positive reactivity (less than 5%) in few tissues. NGS-12-Z1 and NB-27-Z5 did not show significant positive reactivity, indicating no specific binding with 34 normal human tissues.

**Table 4. Summary of Cross Reactivity Assay Results for 34 Human Tissues**

| Number | Organ | Positive Control | Negative Control | BMK1 | BMK2 | NGS-12-Z1 | NGS-16-Z2 | NB-27-Z5 |
|---|---|---|---|---|---|---|---|---|
| 1 | Tonsil | Strong positive for majority of lymphocytes | - | - | Weak positive for a small number of lymphocytes | Weak positive for a small number of lymphocytes | - | - |
| 2 | Liver | - | - | - | - | - | - | - |
| 3 | Thyroid | - | - | - | - | - | - | - |
| 4 | Ileum | Positive for mucosal cells | - | - | - | - | - | - |
| 5 | Cerebellum | Positive for approximate 5% cells in molecular layer | - | - | - | - | - | - |
| 6 | Spleen | Positive for widespread spleen cells | - | - | - | - | - | - |
| 7 | Heart | Occasionally positive for Cardiomyocytes | - | - | - | - | - | - |
| 8 | Artery | Rare positive for stroma | - | - | - | - | - | - |
| 9 | Parathyroid Gland | - | - | - | - | - | - | - |
| 10 | Spinal Cord | Occasionally positive for nerve cells | - | - | - | - | - | - |
| 11 | Fallopian Tube | - | - | Spindle shaped fibrocytes positive (5%) | - | - | - | - |
| 12 | Duodenum | - | - | - | - | - | - | - |
| 13 | Pituitary | - | - | - | - | - | - | - |
| 14 | Stomach | - | - | - | - | - | - | - |
| 15 | Testis | Positive for seminiferous epithelium | - | Occasionally positive for seminiferous epithelium | Rare positive for seminiferous epithelium | - | Rare positive for seminiferous epithelium | - |
| 16 | Colon | - | - | - | - | - | - | - |
| 17 | Bladder | Occasionally positive for epithelium | - | - | - | - | - | - |
| 18 | Uterine Body | - | - | - | - | - | - | - |
| 19 | Lymph Node | Occasionally positive for lymphocytes | - | - | Occasionally positive for lymphocytes | - | - | - |
| 20 | Skin | Rare positive for stromal cells | - | - | - | - | - | - |
| 21 | Placenta | Occasionally positive for chorion tissue | - | - | - | - | - | - |
| 22 | Eye | - | - | - | - | - | - | - |
| 23 | Pancreas | - | - | - | - | - | - | - |
| 24 | Kidney | - | - | - | - | - | - | - |
| 25 | Breast | - | - | - | - | - | - | - |
| 26 | Adrenal Gland | - | - | - | - | - | - | - |
| 27 | Vein | Occasionally positive for stromal cells | - | - | - | - | - | - |
| 28 | Ureter | - | - | - | - | - | - | - |
| 29 | Cervix | - | - | - | - | - | - | - |
| 30 | Prostate | - | - | - | - | - | - | - |
| 31 | Ovary | Occasionally positive for cortex | - | Rare (2%) fibrocyte staining, rare (1%) lymphocytestaining | - | - | - | - |
| 32 | Skeletal Muscle | - | - | - | - | - | - | - |
| 33 | Lung | - | - | - | - | - | - | - |
| 34 | Brain | - | - | - | - | - | - | - |

### Example 9. Summary of sequences

**Table 5. Summary of Antibody Sequences**

| **Number** | **Amino Acid Sequence of Antibody** |
|---|---|
| NB-27 | EVQLVESGGGLAQPGGSLRLSCAASGSTLDPFVIGWFRQAPGKERETVACINR SRTSYADSVKGRFTISRDNAKNMVYLQMNSLKPEDTAVYYCAASPDKPFYTD QTMCNKHYYGYWTQGTQVTVSS（SEQ ID NO:27） |
| NB-57 | EVQLVESGGGLAQSGGSLRLSCAVSGSTLDPFVIGWFRQAPGKERETVSCINR NRASYADGVKGRFTISRDTAKNMVYLQMNSLKPEDTAVYYCAASPDKAFYT DQTMCNIHYYGYWAQGTQVTVSS（SEQ ID NO:28） |
| NB-783 | QVQLVESGGGLAQSGGSLRLSCAVSGSTLDPFVIGWFRQAPGKERETVSCINR NRASYADGVKGRFTISRDTAKNMVYLQMNSLKPEDTAVYYCAASPDKAFYT DQTMCNIHYYGYWAQGTLVTVSS（SEQ ID NO:29） |
| NB-784 | EVQLVESGGGLVQPGGSLRLSCAVSGLNFGIYDFTWGRQAPGKGLEWIATMN PDGDITNYVDSVKGRFTISRDNANESLYLQMNSLKPEDTALYYCALGPERYFS IYATRGQGTLVTVSS（SEQ ID NO:30） |
| NB-879 | QVQLVESGGGLVQPGGSLRLSCASSGDTLESFAVGWFRQAPGKEREELACIGR TGEFTDYPGSVKGRFTITRDNAKNTVYLEMVNLKAEDTGVYYCAAERLVFHL CSLSEYDYDTWGQGTQVTVSS（SEQ ID NO:31） |
| NGS-08 | EVQLVESGGGLVQPGGSLRLSCVASGFTLDDHAIGWFRQAPGKAREGVSCISS SGRTTYLLSVKGRFTITRDNAKNTAYLQMNRLQPEDTAVYYCASVPTLGTAC LMTHGMRSDWGQGTQVTVSS（SEQ ID NO:32） |
| NGS-12 | QVQLVESGGGLVQPGGSLRLSCAVSGFSSDYYDIGWFRQDPEKEREMVSCIRS SGGSTKYADSVKGRFTISRDNAKNTVYLQMNSLEPEDTAVYYCGLAPISPVH AVCNQHYFGYWGQGTRVTVSS（SEQ ID NO:33） |
| NGS-13 | QVQLVESGGGLVQPGGSLRLSCAASGFTLDDQAIGWFRQAPGKEREGVACISS SGRGTYLLSVKGRFTVTRDNAKNTVYLQMNRLQPEDTAVYYCASVPAVQTM CLMTHASRDDWGQGTQVTVSS（SEQ ID NO:34） |
| NGS-16 | QVQLVESGGGLVQPGGSLRLSCEASGGTLDYWSIGWFRQAPGKEREEVSCISG TGLRTNYADSVKGRFTVSRDNSRNSVILQMNSLKPEDTGVYYCTGKLLHPSL WSACANEYDLWGQGTQVTVSS（SEQ ID NO:35） |
| NGS-12-z1 | EVQLVESGGGLVQPGGSLRLSCAASGFSSDYYDIGWFRQAPGKEREMVSCIRS SGGSTKYADSVKGRFTISRDNSKNTVYLQMNSLRAEDTAVYYCGLAPISPVH AVCNQHYFGYWGQGTRVTVSS（SEQ ID NO:36） |
| NGS-12-z2 | EVQLVESGGGLVQPGGSLRLSCAASGFSSDYYDIGWFRQDPGKEREMVSCIRS SGGSTKYADSVKGRFTISRDNSKNTVYLQMNSLRAEDTAVYYCGLAPISPVH AVCNQHYFGYWGQGTRVTVSS（SEQ ID NO:37） |
| NGS-12-z3 | EVQLVESGGGLVQPGGSLRLSCAASGFSSDYYDIGWFRQAPEKEREMVSCIRS SGGSTKYADSVKGRFTISRDNSKNTVYLQMNSLRAEDTAVYYCGLAPISPVH AVCNQHYFGYWGQGTRVTVSS（SEQ ID NO:38） |
| NGS-12-z4 | EVQLVESGGGLVQPGGSLRLSCAASGFSSDYYDIGWFRQDPEKEREMVSCIRS SGGSTKYADSVKGRFTISRDNSKNTVYLQMNSLRAEDTAVYYCGLAPISPVH AVCNQHYFGYWGQGTRVTVSS（SEQ ID NO:39） |
| NGS-12-z5 | EVQLVESGGGLVQPGGSLRLSCAASGFSSDYYDIGWFRQDPEKEREMVSCIRS SGGSTKYADSVKGRFTISRDNSKNTVYLQMNSLEAEDTAVYYCGLAPISPVH AVCNQHYFGYWGQGTRVTVSS（SEQ ID NO:40） |
| NGS-16-z1 | EVQLVESGGGLVQPGGSLRLSCAASGGTLDYWSIGWFRQAPGKEREEVSCISG TGLRTNYADSVKGRFTISRDNSKNTVYLQMNSLRAEDTAVYYCTGKLLHPSL WSACANEYDLWGQGTQVTVSS（SEQ ID NO:41） |
| NGS-16-z2 | EVQLVESGGGLVQPGGSLRLSCAASGGTLDYWSIGWFRQAPGKEREEVSCISG TGLRTNYADSVKGRFTISRDNSKNTVILQMNSLRAEDTAVYYCTGKLLHPSL WSACANEYDLWGQGTQVTVSS（SEQ ID NO:42） |
| NGS-16-z3 | EVQLVESGGGLVQPGGSLRLSCAASGGTLDYWSIGWFRQAPGKEREEVSCISG TGLRTNYADSVKGRFTISRDNSKNTVYLQMNSLRAEDTGVYYCTGKLLHPSL WSACANEYDLWGQGTQVTVSS（SEQ ID NO:43） |
| NGS-16-z4 | EVQLVESGGGLVQPGGSLRLSCAASGGTLDYWSIGWFRQAPGKEREEVSCISG |
| | TGLRTNYADSVKGRFTISRDNSKNTVILQMNSLRAEDTGVYYCTGKLLHPSL WSACANEYDLWGQGTQVTVSS（SEQ ID NO:44） |
| NGS-16-z5 | EVQLVESGGGLVQPGGSLRLSCAASGGTLDYWSIGWFRQAPGKEREEVSCISG TGLRTNYADSVKGRFTVSRDNSKNTVILQMNSLRAEDTGVYYCTGKLLHPSL WSACANEYDLWGQGTQVTVSS（SEQ ID NO:45） |
| NGS-16-z6 | EVQLVESGGGLVQPGGSLRLSCAASGGTLDYWSIGWFRQAPGKEREEVSCISG TGLRTNYADSVKGRFTVSRDNSKNSVILQMNSLRAEDTGVYYCTGKLLHPSL WSACANEYDLWGQGTQVTVSS（SEQ ID NO:46） |
| NGS-16-z7 | EVQLVESGGGLVQPGGSLRLSCEASGGTLDYWSIGWFRQAPGKEREEVSCISG TGLRTNYADSVKGRFTISRDNSKNTVYLQMNSLRAEDTAVYYCTGKLLHPSL WSACANEYDLWGQGTQVTVSS（SEQ ID NO:47） |
| NGS-16-z8 | EVQLVESGGGLVQPGGSLRLSCEASGGTLDYWSIGWFRQAPGKEREEVSCISG TGLRTNYADSVKGRFTISRDNSKNTVILQMNSLRAEDTAVYYCTGKLLHPSL WSACANEYDLWGQGTQVTVSS（SEQ ID NO:48） |
| NGS-16-z9 | EVQLVESGGGLVQPGGSLRLSCEASGGTLDYWSIGWFRQAPGKEREEVSCISG TGLRTNYADSVKGRFTISRDNSKNTVYLQMNSLRAEDTGVYYCTGKLLHPSL WSACANEYDLWGQGTQVTVSS（SEQ ID NO:49） |
| NGS-16-z 10 | EVQLVESGGGLVQPGGSLRLSCEASGGTLDYWSIGWFRQAPGKEREEVSCISG TGLRTNYADSVKGRFTISRDNSKNTVILQMNSLRAEDTGVYYCTGKLLHPSL WSACANEYDLWGQGTQVTVSS（SEQ ID NO:50） |
| NGS-16-z11 | EVQLVESGGGLVQPGGSLRLSCEASGGTLDYWSIGWFRQAPGKEREEVSCISG TGLRTNYADSVKGRFTVSRDNSKNTVILQMNSLRAEDTGVYYCTGKLLHPSL WSACANEYDLWGQGTQVTVSS（SEQ ID NO:51） |
| NB-27-z1 | EVQLVESGGGLVQPGGSLRLSCAASGSTLDPFVIGWFRQAPGKERETVSCINR SRTSYADSVKGRFTISRDNSKNTVYLQMNSLRAEDTAVYYCAASPDKPFYTD QTMCNKHYYGYWTQGTQVTVSS（SEQ ID NO:52） |
| NB-27-z2 | EVQLVESGGGLVQPGGSLRLSCAASGSTLDPFVIGWFRQAPGKERETVSCINR SRTSYADSVKGRFTISRDNSKNMVYLQMNSLRAEDTAVYYCAASPDKPFYTD QTMCNKHYYGYWTQGTQVTVSS（SEQ ID NO:53） |
| NB-27-z3 | EVQLVESGGGLVQPGGSLRLSCAASGSTLDPFVIGWFRQAPGKERETVSCINR SRTSYADSVKGRFTISRDNAKNTVYLQMNSLRAEDTAVYYCAASPDKPFYTD |
| | QTMCNKHYYGYWTQGTQVTVSS（SEQ ID NO:54） |
| NB-27-z4 | EVQLVESGGGLVQPGGSLRLSCAASGSTLDPFVIGWFRQAPGKERETVACINR SRTSYADSVKGRFTISRDNSKNTVYLQMNSLRAEDTAVYYCAASPDKPFYTD QTMCNKHYYGYWTQGTQVTVSS（SEQ ID NO:55） |
| NB-27-z5 | EVQLVESGGGLVQPGGSLRLSCAASGSTLDPFVIGWFRQAPGKERETVACINR SRTSYADSVKGRFTISRDNSKNMVYLQMNSLRAEDTAVYYCAASPDKPFYTD QTMCNKHYYGYWTQGTQVTVSS（SEQ ID NO:56） |
| NB-27-z6 | EVQLVESGGGLVQPGGSLRLSCAASGSTLDPFVIGWFRQAPGKERETVACINR SRTSYADSVKGRFTISRDNAKNTVYLQMNSLRAEDTAVYYCAASPDKPFYTD QTMCNKHYYGYWTQGTQVTVSS（SEQ ID NO:57） |
| NB-27-z7 | EVQLVESGGGLVQPGGSLRLSCAASGSTLDPFVIGWFRQAPGKERETVACINR SRTSYADSVKGRFTISRDNAKNMVYLQMNSLRAEDTAVYYCAASPDKPFYTD QTMCNKHYYGYWTQGTQVTVSS（SEQ ID NO:58） |
| NB-27-z8 | EVQLVESGGGLVQPGGSLRLSCAASGSTLDPFVIGWFRQAPGKERETVSCINR SRTSYADSVKGRFTISRDNSKNTLYLQMNSLRAEDTAVYYCAASPDKPFYTD QTMCNKHYYGYWTQGTQVTVSS（SEQ ID NO:59） |
| NB-27-z9 | EVQLVESGGGLVQPGGSLRLSCAASGSTLDPFVIGWFRQAPGKGRETVACINR SRTSYADSVKGRFTISRDNAKNMVYLQMNSLRAEDTAVYYCAASPDKPFYTD QTMCNKHYYGYWTQGTQVTVSS（SEQ ID NO:60） |
| NB-27-z10 | EVQLVESGGGLVQPGGSLRLSCAASGSTLDPFVIGWFRQAPGKGRETVSCINR SRTSYADSVKGRFTISRDNSKNTVYLQMNSLRAEDTAVYYCAASPDKPFYTD QTMCNKHYYGYWTQGTQVTVSS（SEQ ID NO:61） |
| NB-879-z1 | EVQLVESGGGLVQPGGSLRLSCAASGDTLESFAVGWFRQAPGKEREELSCIGR TGEFTDYPGSVKGRFTISRDNSKNTVYLQMNSLRAEDTAVYYCAAERLVFHL CSLSEYDYDTWGQGTQVTVSS（SEQ ID NO:62） |
| NB-879-z2 | EVQLVESGGGLVQPGGSLRLSCAASGDTLESFAVGWFRQAPGKEREELSCIGR TGEFTDYPGSVKGRFTISRDNAKNTVYLQMNSLRAEDTAVYYCAAERLVFHL CSLSEYDYDTWGQGTQVTVSS（SEQ ID NO:63） |
| NB-879-z3 | EVQLVESGGGLVQPGGSLRLSCAASGDTLESFAVGWFRQAPGKEREELSCIGR TGEFTDYPGSVKGRFTITRDNSKNTVYLQMNSLRAEDTAVYYCAAERLVFHL CSLSEYDYDTWGQGTQVTVSS（SEQ ID NO:64） |
| NB-879-z4 | EVQLVESGGGLVQPGGSLRLSCAASGDTLESFAVGWFRQAPGKEREELACIG RTGEFTDYPGSVKGRFTISRDNSKNTVYLQMNSLRAEDTAVYYCAAERLVFH LCSLSEYDYDTWGQGTQVTVSS（SEQ ID NO:65） |
| NB-879-z5 | EVQLVESGGGLVQPGGSLRLSCAASGDTLESFAVGWFRQAPGKEREELACIG RTGEFTDYPGSVKGRFTISRDNAKNTVYLQMNSLRAEDTAVYYCAAERLVFH LCSLSEYDYDTWGQGTQVTVSS（SEQ ID NO:66） |
| NB-879-z6 | EVQLVESGGGLVQPGGSLRLSCAASGDTLESFAVGWFRQAPGKEREELACIG RTGEFTDYPGSVKGRFTITRDNSKNTVYLQMNSLRAEDTAVYYCAAERLVFH LCSLSEYDYDTWGQGTQVTVSS（SEQ ID NO:67） |
| NB-879-z7 | EVQLVESGGGLVQPGGSLRLSCAASGDTLESFAVGWFRQAPGKEREELACIG RTGEFTDYPGSVKGRFTITRDNAKNTVYLQMNSLRAEDTAVYYCAAERLVFH LCSLSEYDYDTWGQGTQVTVSS（SEQ ID NO:68） |
| NB-879-z11 | EVQLVESGGGLVQPGGSLRLSCAASGDTLESFAVGWFRQAPGKEREEVACIG RTGEFTDYPGSVKGRFTISRDNSKNTVYLQMNSLRAEDTAVYYCAAERLVFH LCSLSEYDYDTWGQGTQVTVSS（SEQ ID NO:69） |
| NB-879-z12 | EVQLVESGGGLVQPGGSLRLSCAASGDTLESFAVGWFRQAPGKEREEVACIG RTGEFTDYPGSVKGRFTISRDNAKNTVYLQMNSLRAEDTAVYYCAAERLVFH LCSLSEYDYDTWGQGTQVTVSS（SEQ ID NO:70） |
| NB-879-z13 | EVQLVESGGGLVQPGGSLRLSCAASGDTLESFAVGWFRQAPGKEREEVACIG RTGEFTDYPGSVKGRFTITRDNSKNTVYLQMNSLRAEDTAVYYCAAERLVFH LCSLSEYDYDTWGQGTQVTVSS（SEQ ID NO:71） |
| NB-879-z14 | EVQLVESGGGLVQPGGSLRLSCAASGDTLESFAVGWFRQAPGKEREELACIG RTGEFTDYPGSVKGRFTITRDNSKNTVYLEMNSLRAEDTAVYYCAAERLVFH LCSLSEYDYDTWGQGTQVTVSS（SEQ ID NO:72） |
| NB-879-z15 | EVQLVESGGGLVQPGGSLRLSCASSGDTLESFAVGWFRQAPGKEREELACIGR TGEFTDYPGSVKGRFTITRDNSKNTVYLQMVSLRAEDTAVYYCAAERLVFHL CSLSEYDYDTWGQGTQVTVSS（SEQ ID NO:73） |
| BMK1 | QVQLVESGGGVVQPGRSLRLSCAASGFTFSSYTMHWVRQAPGKGLEWVTFIS YDGNNKYYADSVKGRFTISRDNSKNTLYLQMNSLRAEDTAIYYCARTGWLG PFDYWGQGTLVTVSSGGGGSGGGGSGGGGSGGGGASEIVLTQSPGTLSLSPG ERATLSCRASQSVGSSYLAWYQQKPGQAPRLLIYGAFSRATGIPDRFSGSGSG |
| | TDFTLTISRLEPEDFAVYYCQQYGSSPWTFGQGTKVEIK（SEQ ID NO:75） |
| BMK2 | QVQLVESGGGLVQPGGSLRLSCAASGYIYSAYCMGWFRQAPGKGLEGVAAI YIGGGSTYYADSVKGRFTISRDNSKNTLYLQMNSLRAEDTAVYYCAADVIPTE TCLGGSWSGPFGYWGQGTLVTSS （SEQ ID NO:76） |
| Isotype | See SEQ ID NO:2 in CN 106146653A |
| IgG4 Fc | YGPPCPPCPAPEFLGGPSVFLFPPKPKDTLMISRTPEVTCVVVDVSQEDPEVQF NWYVDGVEVHNAKTKPREEQFNSTYRVVSVLTVLHQDWLNGKEYKCKVSN KGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGK（SEQ ID NO:74） |

**Table 6. Summary of Antibody CDR Sequences**

| **Antibody Number** | **CDR1** | **SE Q ID NO** | **CDR2** | **SE Q ID NO** | **CDR3** | **SE Q ID NO** |
|---|---|---|---|---|---|---|
| NB-27 | GSTLDPF V | 4 | INRSRT | 11 | AASPDKPFYTDQTMCNKHYYG Y | 19 |
| NB-57 | GSTLDPF V | 4 | INRNRA | 12 | AASPDKAFYTDQTMCNIHYYG Y | 20 |
| NB-783 | GSTLDPF V | 4 | INRNRA | 12 | AASPDKAFYTDQTMCNIHYYG Y | 20 |
| NB-784 | GLNFGIY D | 5 | MNPDGDI T | 13 | ALGPERYFSIYAT | 21 |
| NB-879 | GDTLESF A | 6 | IGRTGEFT | 14 | AAERLVFHLCSLSEYDYDT | 22 |
| NGS-08 | GFTLDDH A | 7 | ISSSGRT | 15 | ASVPTLGTACLMTHGMRSD | 23 |
| NGS-12 | GFSSDYY D | 8 | IRSSGGST | 16 | GLAPISPVHAVCNQHYFGY | 24 |
| NGS-13 | GFTLDDQ | 9 | ISSSGRG | 17 | ASVPAVQTMCLMTHASRDD | 25 |
| | A | | | | | |
| NGS-16 | GGTLDY WS | 10 | ISGTGLRT | 18 | TGKLLHPSLWSACANEYDL | 26 |

## Claims

1. A CTLA-4 binding molecule, comprising an anti-CTLA-4 single domain antibody, wherein the complementarity determining region (CDRs) of the single domain antibody comprise CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3, wherein,
SEQ ID NO: 1 is GX₁X₂X₃X₄X₅X₆X₇, wherein X₁ is S, L, D, F or G, X₂ is T, N or S, and X₃ is L, F or S, X₄ is D, G or E, X₅ is P, I, S, D or Y, X₆ is F, Y, H, Q or W, and X₇ is V, D, A or S,
SEQ ID NO: 2 is X₁X₂X₃X₄X₅X₆X₇X₈, wherein X₁ is I or M, X₂ is N, G, S or R, X₃ is R, P, S or G, X₄ is S, N, D or T, X₅ is R or G, X₆ is T, A, D, E, R, G or L, X₇ is I, F, T, S, G, R or null, and X₈ is T or null,
SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, G or T, X₂ is A, L, S or G, X₃ is S, G, E, V, A or K, X₄ is the P, R or L, X₅ is D, E, L, T, I or A, X₆ is K, R, V, L, S or H, X₇ is P, A, Y, F, G or Q, X₈ is F, H, T, V or S, X₉ is Y, S, L, A, H or M, X₁₀ is T, I, C, A or W, X₁₁ is D, Y, S, L or V, X₁₂ is Q, A, L, M or C, X₁₃ is T, S, N or C, X₁₄ is M, E, H, Q, A or null, X₁₅ is C, Y, G, H, A, N or null, X₁₆ is N, D, M, Y, S, E or null, X₁₇ is K, I, Y, R, F or null, X₁₈ is H, D, S, G or null, X₁₉ is Y, T, D, L or null, X₂₀ is Y or null, X₂₁ is G or null, and X₂₂ is Y or null.

2. The CTLA-4 binding molecule according to claim 1, wherein CDR1 of the single domain antibody comprises the sequence shown in any one of SEQ ID NOs: 4-10, CDR2 comprises the sequence shown in any one of SEQ ID NOs: 11-18, and CDR3 comprises the sequence shown in any one of SEQ ID NOs: 19-26,
preferably, the single domain antibody comprises CDR1, CDR2 and CDR3 shown in any of the following groups a1 to a9:
| Group | CDR1 | CDR2 | CDR3 |
|---|---|---|---|
| a1 | 4 | 11 | 19 |
| a2 | 4 | 12 | 20 |
| a3 | 4 | 12 | 20 |
| a4 | 5 | 13 | 21 |
| a5 | 6 | 14 | 22 |
| a6 | 7 | 15 | 23 |
| a7 | 8 | 16 | 24 |
| a8 | 9 | 17 | 25 |
| a9 | 10 | 18 | 26 |

3. The CTLA-4 binding molecule according to claim 1 or 2, wherein,
the FR region of the single domain antibody comprises the FR region of any VHH selected from SEQ ID NOs: 27-73, and/or
the single domain antibody VHH is as shown in any one of SEQ ID NOs: 27-73, or has at least 80%, 90%, 95%, 96%, 97%, 98%, or 99% identity to amino acid sequence as shown in any one of SEQ ID NOs: 27-73, and/or
the CTLA-4 binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.

4. A polynucleotide, wherein the polynucleotide comprises a sequence selected from:
(1) a coding sequence of the CTLA-4 binding molecule according to any one of claims 1-3;
(2) a complementary sequence of (1);
(3) a 5-50 bp fragment of any sequence of (1) or (2).

5. A nucleic acid construct, wherein the nucleic acid construct comprises the polynucleotide of claim 4, preferably, the nucleic acid construct is a recombinant vector or expression vector.

6. A Phage comprising the CTLA-4 binding molecules according to any one of claims 1-3, preferably, the CTLA-4 binding molecule is displayed on the surface of the phage.

7. A host cell, wherein the host cell:
(1) expresses the CTLA-4 binding molecules according to any one of claims 1-3; and/or
(2) comprises the polynucleotide according to claim 4; and/or
(3) comprises the nucleic acid construct according to claim 5.

8. A method for producing CTLA-4 binding molecules, comprising: culturing the host cell according to claim 7 under conditions suitable for producing the CTLA-4 binding molecules, and optionally purifying the CTLA-4 binding molecules from the culture.

9. A pharmaceutical composition, comprising the CTLA-4 binding molecule according to any one of claims 1-3, the polynucleotide according to claim 4, the nucleic acid construct according to claim 5, the phage according to claim 6 or the host cell according to claim 7, and pharmaceutically acceptable excipients, preferably, the pharmaceutical composition is used for treating cancer.

10. Use of the CTLA-4 binding molecule according to any one of claims 1-3 in the preparation of a medicament for the prevention or treatment of a cancer.

11. A kit for detecting CTLA-4, which is used to evaluate the therapeutic effect of a medicament or diagnose cancer, wherein the kit comprises the CTLA-4 binding molecule according to any one of claims 1-3, the polynucleotide according to claim 4, the nucleic acid construct according to claim 5, the phage according to claim 6 or the host cell according to claim 7,
preferably, the kit further comprises a reagent for detecting the binding of CTLA-4 to a single domain antibody, an antibody, or an antigen binding fragment thereof,
more preferably, the reagent is a reagent that detects the binding by enzyme-linked immunosorbent assay.

12. A non diagnostic method for detecting the presence of CTLA-4 in a sample, wherein the method comprises: incubating a CTLA-4 binding molecule according to any one of claims 1-3 with the sample, and detecting the binding of CTLA-4 to a single domain antibody, antibody, or antigen binding fragment thereof, thereby determining the presence of PD-1 in the sample.

13. Use of the CTLA-4 binding molecule according to any one of claims 1-3 in the preparation of a kit for detecting CTLA-4 in samples, evaluating the therapeutic effect of a medicament, or diagnosing a cancer.
